(19) Europäisches Patentamt / European Patent Office / Office européen des brevets

(11) **EP 4 666 934 A1**

(12) **EUROPEAN PATENT APPLICATION**

(43) Date of publication:
24.12.2025 Bulletin 2025/52

(21) Application number: 24183378.9

(22) Date of filing: **20.06.2024**

(51) International Patent Classification (IPC):
*A61B 5/00* (2006.01)  *A61B 5/339* (2021.01)
*A61B 5/24* (2021.01)  *G06T 19/20* (2011.01)
*G16H 50/50* (2018.01)  *A61B 5/318* (2021.01)

(52) Cooperative Patent Classification (CPC):
**A61B 5/339; A61B 5/24; A61B 5/318; A61B 5/743;
G06T 19/20; G16H 50/50**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• ZHANG, Yuyang
  Eindhoven (NL)
• WU, Jikun
  Eindhoven (NL)
• YANG, Chao
  5656AG Eindhoven (NL)
• CHEN, Chaobin
  Eindhoven (NL)

(74) Representative: **Philips Intellectual Property &
Standards
High Tech Campus 52
5656 AG Eindhoven (NL)**

(54) **PROVIDING INFORMATION ON ELECTRICAL MEASURES OF THE HEART**

(57)    A mechanism for visually representing a heart. Measures of electrical activity of the heart are received. Each measure is associated with a different portion or part of a virtual model of the heart. A position of locations in each corresponding portion/part of the virtual model is/are modified responsive to the corresponding measure of electrical activity.

FIG. 2

EP 4 666 934 A1

**Description**

FIELD OF THE INVENTION

The present disclosure relates to the field of cardiac monitoring, and in particular to electrical measures of the heart.

BACKGROUND OF THE INVENTION

There is an increasing interest in and reliance upon cardiac monitoring within the medical profession. In particular, it is becoming increasingly important to monitor electrical activity of the heart (e.g., using electrocardiography) in order to monitor a condition of the heart and identify any erroneous or undesirable behavior.
A common technique for performing heart monitoring is an electrocardiography technique, which will produce an electrocardiogram (ECG). Typically, this involves positioning a number of electrodes on the surface of a subject's skin, and defining a number of voltages that represent different "angles" of the electrical potential of the heart. These angles are more commonly known as leads. One traditional form of electrocardiogram is called a 12-lead ECG.
There is an ongoing interest in improving the interpretability of electrocardiograms, and reducing a risk of misdiagnosis or incorrect assessment.

SUMMARY OF THE INVENTION

The invention is defined by the claims.
According to examples in accordance with an aspect of the invention, there is provided a computer-implemented method for providing a visual representation of a heart of a subject.
The computer-implemented method comprises: receiving a plurality of different electrical measures responsive to electrical activity of the heart; receiving a virtual model of the heart, wherein each electrical measure is associated with a respective portion of the virtual model; for each electrical measure, modifying a position of the respective portion of the virtual model, with respect to the remainder of the virtual model, responsive to the electrical measure; and providing a visual representation of the virtual model at an output user interface.
It is herein proposed to alter an appearance of a virtual model of the heart to reflect changes in different areas of the heart that are associated with different electrical measures. In particular, it has been recognized that it is possible to associate different electrical measures with different parts of the heart. By modifying the position of these parts of the heart responsive to the electrical measures, it is possible to effectively model changes in the electrical activity of the heart as identified in the electrical measures. This allows for unusual or undesirable electrical measures to be easily identified and associated with their corresponding parts of the heart.
The present disclosure thereby provides a mechanism in which faults at or in particular locations or regions of the heart can be more flagged. In particular, by tying or associating different parts or areas of the heart to different electrical measures, it is possible to more immediately identify failures or errors within the heart.
The proposed approach enables a skilled person to simultaneously view, via looking at a single visual representation of a model of the heart, the (localized) effect of different electrical measures on the heart. This allows the simultaneous overview display of several pieces of medical data.
In some examples, each electrical measure is an electrocardiography voltage of a different cardiography lead. In some examples, each electrocardiography voltage is generated using one or more electrocardiography electrodes positioned on skin of the subject.
Each electrical measure may be a most recently available version of the electrical measure. This facilitates active monitoring of localized effects or conditions of the heart for more responsive and direct monitoring of the subject.
In some examples, for each electrical measure, modifying a position of the portion of the virtual model comprises: for each point of the virtual model in the portion of the virtual model, moving the point of the virtual model in a direction perpendicular to a surface of the virtual model at said point, wherein a distance of the movement of the point is responsive to the electrical measure. This provides a direct modification to the virtual model of the heart that can be readily and intuitively identified by an operator. This movement resembles the movement of a heart and facilitates identification of acute changes identified in the electrical measures.
The distance of the movement of each point may be responsive to a predetermined proportion of the electrical measure.
The virtual model may be a mesh model of the heart. This provides an easily manipulatable model that can be modified and varied according to the electrical measures.
The mesh model may define a plurality of nodes, each representing a different part of the heart. The computer-implemented may further comprise, for each node in a subset of the plurality of nodes of the mesh mode: processing the plurality of different electrical measures to determine a movement of the part of heart represented by the node, which movement results from a movement of the heart indicated in the plurality of different electrical measures; and modifying the position of the node responsive to the determined movement of said node.

More particularly, processing the plurality of different electrical measures may comprise using a cardiac model to model a shape deformation of the heart and determining a movement of the part of the heart responsive to the shape deformation of the heart.

It will be appreciated that different locations about the virtual model effectively represent different parts of the heart. The method may further comprise, for each of a plurality of (such) locations about the virtual model, processing the plurality of different electrical measures to determine a respective movement of the part of heart represented by the location . This respective movement results from a movement of the heart indicated in the plurality of different electrical measures. The method may further comprise modifying the location responsive to the respective movement of each location.

This approach provides a technique for moving or manipulating a location or point of the virtual model to represent a predicted movement of the overall heart. This provides more accurate and relevant identification of heart movement for an individual, e.g., to facilitate comparisons between the movements of different parts of the heart.

In some examples, the step of providing a visual representation of the virtual model at an output user interface comprises visually distinguishing the visual representation of different portions of the heart model, associated with different electrical measures, from one another. This increases an ease of distinguishing any effect indicated in different electrical measures from one another.

In some examples, the step of providing a visual representation of the virtual model at an output user interface comprising visually distinguishing the visual representation of any part of the heart model that contains a portion associated with any electrical measures from any part of the heart model that contains a portion associated with no electrical measures.

In some examples, each of the plurality of electrical measures is sampled at a same or similar point in time.

There is also provided a computer-implemented method for providing a visual representation of a heart of a subject. The computer-implemented method comprises: receiving a sequence of time-dependent data entries, each data entry comprising a plurality of different electrical measures responsive to electrical activity of the heart; and for each time-dependent data entry in turn, performing an instance of any previously described method, wherein the plurality of different electrical measures received in performing the instance of the method comprises the plurality of different electrical measures in the time-dependent data entry.

There is also provided a computer program product comprising computer program code means which, when executed on a computing device having a processing system, cause the processing system to perform all of the steps of the computer-implemented method according to any previously described method.

There is also provided a processing system for providing a visual representation of a heart of a subject. The processing system is configured to: receive a plurality of different electrical measures responsive to electrical activity of the heart; receive a virtual model of the heart, wherein each electrical measure is associated with a respective portion of the virtual model; for each electrical measure, modify a position of the respective portion of the virtual model, with respect to the remainder of the virtual model, responsive to the electrical measure; and provide a visual representation of the virtual model at an output user interface.

There is also provided an output user interface system comprising: the processing system; and the output user interface.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments may be employed;
Fig. 2 is a flowchart illustrating a proposed method; and
Fig. 3 illustrates a virtual model.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for visually representing a heart. Measures of electrical activity of the heart are received. Each measure is associated with a different portion or part of a virtual model of the heart. A position of locations in each corresponding portion/part of the virtual model is/are modified responsive to the corresponding measure of electrical activity.

In the context of the present disclosure, a subject may be a medical subject such as a patient within a medical care facility (e.g., a clinic, a hospital, an ambulance and so on), but may also be an individual, animal or person outside of such environments, e.g., an individual within a home environment.

In the context of the present disclosure, a subset of a group of elements may comprise only some or part (i.e., not all) of the group of elements or the entirety of the group of elements.

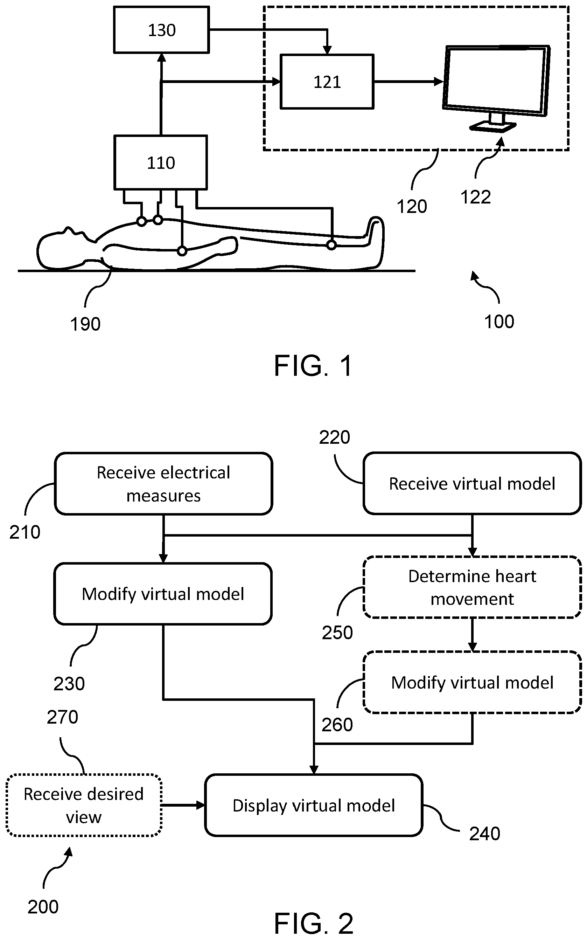

Fig. 1 illustrates a system 100 in which embodiments may be employed. The system comprises a cardiac monitoring system 110 and an output user interface 120, itself an embodiment.

The cardiac monitoring system 110 is configured to (iteratively) generate a plurality of different electrical measures responsive to electrical activity of the heart of a subject 190. For instance, the cardiac monitoring system may comprise an electrocardiogram (ECG) system configured to record the electrical signal(s) produced by the heart.

In this way, each electrical measure may be an electrocardiography voltage of a different cardiography lead. More specifically, each electrocardiography voltage may be generated using one or more electrocardiography electrodes positioned on the skin of the subject.

The function of an ECG system is well known in the art, and a detailed description of such a system is not provided for the sake of conciseness. Nonetheless, it is noted that, typically, an ECG system comprises a plurality of electrodes that are positioned upon the skin surface of the subject 190. A voltage at each electrode is monitored, and used to produce a plurality of different electrical measures. More particularly, the magnitude of the heart's electrical potential across different angles are measured to produce a plurality of lead voltages.

A typical ECG system is a 12-lead ECG system that iteratively produces 12 different electrical measures of the heart (each representing a different view or lead of the heart). These measures typically include 6 measures generated by electrodes positioned on limbs of the subject and 6 measures generated by electrodes positioned on the chest of the subject (e.g., a respective measure produced by each electrode positioned on the chest). Common labels for these leads (and therefore measures) include: I, II, III, aVF, aVL, aVF, V1, V2, V3, V4, V5, and V6.

ECG systems with additional leads (and therefore electrical measures) are known. Other forms of cardiac monitoring systems 110 and/or ECG systems are also known in the art.

The output user interface 120 comprises a processing system 121 (itself an embodiment of the proposed approach) and an output user interface 122.

The processing system 121 is configured to receive the plurality of different electrical measures, e.g., directly from the cardiac monitoring system 110 or from a memory 130 storing information produced by the cardiac monitoring system 110. The processing system 121 processes the plurality of different electrical measures, and controls the output user interface 122 to provide a visual representation of the plurality of different electrical measures.

Each of the plurality of electrical measures received in this way may have been sampled at a same or similar point in time by the cardiac monitoring system.

The output user interface 122 may, for instance, comprise a screen that displays a visual representation of the plurality of different electrical measures. In some examples, the output user interface 122 comprises a printer that provides a physical printout providing a visual representation of the plurality of different electrical measures.

Historically, the processing system simply plots each electrical measure on a respective graph. The processing system will iteratively receive a different plurality of the different electrical measures and, for each iteration, plot a different electrical measure on its respective graph. Thus, each electrical measure is associated with a different graph. Each graph may, for instance, be a physical graph (e.g., a printout) or a virtual graph (e.g., a graph representation of the graph on a display or screen).

The present disclosure provides an alternative technique to representing the plurality of different electrical measures at the output user interface. In particular, the present disclosure proposes to modify a virtual model of the heart responsive to the electrical measures. This provides a clinician with information that directly ties each electrical measure to a position at the heart. This allows the clinician to more immediately understand the effect that a particular electrical measure has upon the shape of the heart, providing the clinician with additional information that was not previously available to them.

The present disclosure therefore proposes a variant to the processing system 121, e.g., for use in the system 100 outlined above.

Fig. 2 is a flowchart illustrating a method 200 performed by a processing system, such as the processing system previously described.

The method comprises a step 210 of receiving a plurality of different electrical measures responsive to electrical activity of the heart. As previously explained, step 210 may be performed by directly receiving the plurality of different electrical measures from a cardiac monitoring system (that produces the electrical measures) or from a storage/memory, which stores the electrical measures produced by the cardiac monitoring system.

Suitable examples of electrical measures, such as those produced by an ECG system, have been previously described.

Preferably, each electrical measure is a most recently available version of the electrical measure. In this way, the method is able to perform real-time updates to the representation of the plurality of different electrical measures at the output interface.

The method 200 also comprises a step 220 of receiving a virtual model of the heart, wherein each electrical measure is associated with a respective portion of the virtual model.

The virtual model may be a generic and/or predefined model, e.g., a population average model, or a subject-specific model. Approaches for producing a subject-specific model of a heart of a subject are well known in the art, such as those that perform segmentation of imaging data of the heart (e.g., employing an approach disclosed by WO 2017/109662 A1) and/or approaches that perform mapping of the heart using data produced by an interventional device within the heart (such as the EPD Solutions navigation system provided by Philips ®, named the KODEX-EPD ® system).

Virtual models of anatomical structures are well known in the art. A typical virtual model may be embodied as a mesh model. A mesh model defines a plurality of vertices (also known as points or nodes), edges and/or faces that define the shape of the virtual model.

The virtual model may be received from a memory or from a virtual model generator. The virtual model generator may generate the model according to any above described approach.

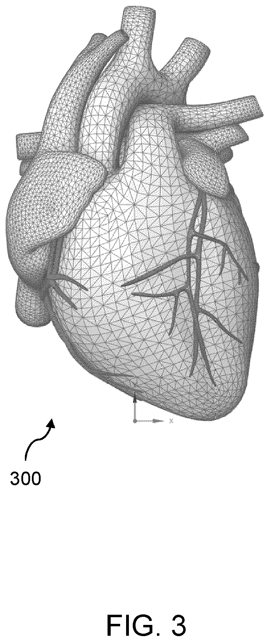

Fig. 3 illustrates one example of a virtual model 300 of the heart. The virtual model is embodied as a mesh model, specifically a triangular mesh that defines a plurality of different nodes/vertices. A surface can be laid over the triangular mesh to represent the shape of the anatomical structure.

Each electrical measure is associated with a respective portion of the virtual model. In particular, each electrical measure will be naturally associated with a different part or portion of the heart of the subject.

For instance, if an electrical measure represents a voltage measured at a particular electrode, then the known or expected position of the electrode can be associated with a known portion of the virtual model. For instance, if it is expected that an electrode that produced an electrical measure will be located above a particular portion of the heart, then the electrical measure can be associated with a portion of the virtual model that represents said particular portion of the heart.

In some examples, if an electrical measure represents a voltage measured at a particular electrode, then the known or expected position of the electrode can be associated with a portion of the virtual model that represents a region of the heart closest to the known or expected position of the electrode.

As an example, consider the scenario in which each electrical measure is an electrical measure for a respective lead of a 12-lead ECG system. Each lead of a 12-lead ECG system has a predefined label that is well established in the art. Table 1 illustrates the relationship between the label of the lead (and its associated electrical measure) and the area of the heart to which the electrical measure is responsive. It is possible to associate each area of the heart with a corresponding portion of the virtual model of the heart. This provides an example of how to associate an electrical measure with a particular portion or area of a virtual model of the heart.

TABLE 1

| Lead Label | Affected Area |
| --- | --- |
| V1, V2 | Septal |
| V3, V4 | Anterior |
| V5, V6 | Apical |
| I, aVL | Lateral |
| II, III, aVL | Inferior |

The data used to produce Table 1 is found in Rafla, Samir, and Amr Kamal. "Localization of the occluded vessel in acute myocardial infarction." J Cardiol Cardiovasc Med 5 (2020): 029-33.

Other approaches and techniques for associating an electrical measure of the heart with a particular portion of a virtual model of the heart will be readily apparent to the skilled person.

Turning back to Fig. 2, the method 200 also comprises a step 230 of, for each electrical measure, modifying a position of the respective portion of the virtual model, with respect to the remainder of the virtual model, responsive to the electrical measure. In this way, the virtual model changes or is modified to reflect changes in the electrical measure(s). This provides a direct representation, on the model of the heart, of changes in the electrical measures of the heart.

The method 200 also comprises a step 240 of providing a visual representation of the virtual model at an output user interface. Approaches for performing step 240 will be readily apparent to the skilled person, and may comprise rendering the virtual model to produce display data and controlling the output user interface using the display data to provide a visual representation of the virtual model.

It will be appreciated that method 200 may be iteratively repeated, e.g., to iteratively update the virtual model with the latest available data for the electrical measures and/or to step through a sequence of pluralities of electrical measures (e.g., stored data).

Thus, there may be a sequence of time-dependent data entries, each comprising a set of electrical measures (i.e., an instance of a plurality of electrical measures). The sequence represents different values for the electrical measures as measured over a period of time.

Thus, there is provided a computer-implemented method comprising: receiving a sequence of time-dependent data entries. Each data entry comprises a plurality of different electrical measures responsive to electrical activity of the heart. For each time-dependent data entry in turn, an instance of method 200 may be performed. In each instance, the plurality of different electrical measures received in performing the instance of the method comprises the plurality of different electrical measures in the time-dependent data entry.

In this way, the virtual model is able to represent the dynamics (shape change) over time. This advantageously provides additional information about the shape of the heart that was not previously available from the ECG traces alone.

One approach to performing step 230 is hereafter described.

In this approach, step 230 comprises (for each electrical measure) moving each point in the corresponding portion of the virtual model in a direction perpendicular to a surface of the virtual model at said point. A distance of the movement of the point may be responsive to the electrical measure.

In particular, consider a scenario in which the virtual model is a mesh model that defines a plurality of nodes. Each node is associated with a respective location or position in a three-dimensional space. Step 230 may comprise, for each electrical measure, defining a new location or position for each node within the three-dimensional space using the electrical measure. In this way, the position of each node is modified.

In an extremely simple example, step 230 may comprise defining a new location $D_i$ of a node i using the following equation:

$$D_i = T_i + P_I = \sum_{m=0}^{m=n} \vec{v_i} \times k_T(m)V_j(m) + P_i \qquad (1)$$

where $P_i$ is the existing location of the node i, $V_j(m)$ is the m-th electrical measure associated with a portion of the virtual model into which the node i falls, n is the number of electrical measures into whose associated portion of the virtual model the node i falls, $k_T$ is a weighting or modifier for the localized manipulation and $\vec{v_l}$ is a direction vector that is normal or perpendicular to the surface of the virtual model at the (existing) location of the node i. $T_i$ represents the acute modification to the location of the node i as a result of the electrical measure(s).

If only equation (1) is applied for each node i, then it will be appreciated that a node that does not fall within a portion of the virtual model (associated with an electrical measure) will undergo no movement. Thus, only an incomplete subset of the nodes of the virtual model will undergo a movement.

It is readily possible to define a direction vector that is normal or perpendicular to the surface of the virtual model at a particular node using established techniques, such as those put forward by A. Ubach, C. Estruch, and J. Garcia-Espinosa, "On the interpolation of normal vectors for triangle meshes," International Journal for Numerical Methods in Engineering, vol. 96, no. 4, pp. 247-268, Sep. 2013, doi: 10.1002/nme.4567.

In a more complex example, each electrical measure m is associated with its own direction vector $\overrightarrow{v_{LJ}}(m)$. This may, for instance, be an estimation of the angle of the electrical measure relative to the movement center of the heart. In particular, if an electrical measure is associated with a known or expected location with respect to the heart (e.g., a known location of an electrode), then the direction vector $\overrightarrow{v_{LJ}}(m)$ may represent a direction from the center of the heart to the known/expected location for the electrical measure.

In this example, step 230 may comprise defining a new location $D_i$ for each node i within the portion(s) of the model associated with electrical measure(s) $V_j(m)$ using the following formula:

$$D_i = T_i + P_I = \sum_{m=0}^{m=n} \frac{\overrightarrow{v_{L_j}}(m) \cdot \overrightarrow{v_i}}{|\overrightarrow{v_i}|} \times k_T V_j + P_i \qquad (2)$$

The weighting $k_T(m)$ listed in Equations (1) and (2) is used to control the influence of each electrical measure on each node. In different node locations, the movement amplitude and tendency are naturally different due to the heart's muscle and its structure, which can be indicated or influenced using the weighting $k_T(m)$. The value(s) for the weighting(s) may be referenced from a real beating heart.

Of course, if equation (2) is used, then it will be appreciated that a node that does not fall within a portion of the virtual model (associated with an electrical measure) will undergo no movement. Thus, only an incomplete subset of the nodes of the virtual model will undergo a movement.

For improved similarity to a real heart, thereby improving an understanding of any undesirable effect on the heart, it is possible to determine a movement of the heart which can be used to control the virtual model. In particular, each point of the virtual model may be controlled to resemble or replicate a movement of the heart as indicated in the plurality of electrical measures.

Thus, method 200 may further comprise a step 250, for each of a plurality of locations about the virtual model, processing the plurality of different electrical measures to determine a respective movement of part of heart, represented by the location of the virtual model, resulting from a movement of the heart indicated in the plurality of different electrical measures. The method may further comprise a step 260 of modifying the location responsive to the respective movement of each location. Step 260 may be at least partially integrated into step 230.

This approach aims to simulate a cardiac movement $C_i$ resulting from a cardio cycle of the heart, which provides an approach for performing movement control as a baseline of the heart's movement.

In some examples, each of the plurality of locations is a different node i of a mesh model acting as the virtual model. Equation 3 illustrates one example approach for determining a modification to a position of an existing node i of the virtual model.

$$C_i = SR\left(V_j, \overrightarrow{v_i}, P_i\right).k_C \qquad (3)$$

In a simple model, SR(.) could be a function having an output value that changes with the values of the electrical measures Vj along a direction normal or perpendicular to the surface of the virtual model at the (existing) location of the node i (i.e., the value of $P_i$ is ignored and $k_c = 1$). However, it is recognized that the influence of a cardiac movement on an electrical measure (and therefore a reversal of the same) will drastically change depending on the node i location $P_i$. This difference can be defined using a modifier $k_c$ for the node i.

In particular SR(.) may represent a function that modifies the node(s) of the virtual model according to a normal sinus rhythm. In this way, modifying the position of the node using SR(.) may effectively act to modify the position of each node to resemble or replicate a standard or baseline sinus rhythm of the heart. A predefined function such as SR (.) may effectively include a definition of how exactly each node should move based on a given amplitude extracted from voltage information. More particularly, the function SR(.) may be adapted to process all available electrical measure to determine an expected movement of the i-th node of the heart. One example of a suitable algorithm for functioning as SR(.) makes use of the ST/AR algorithm developed by Philips [®].

In one working example, the function SR(.) may be configured to extract cardio dynamic information and/or PQRST waves from the plurality of different electrical measures. This cardio dynamic information may be used, as part of the function SR(.), to generate a dynamic cardio cycle model of the heart from a predefined sinus rhythm model or function, which defines how much each node should move based on the cardio dynamic information and/or PQRST waves.

More particularly, the function SR(.) may effectively determine a shape deformation of the heart (e.g., a change in shape of the heart) by processing the plurality of different electrical measures and using a predefined model of heart movement. Of course, if the electrical measures do not follow a conventional or expected sinus rhythm (e.g., there are no PQRST waves to be recognized in a series of pluralities of electrical measures), then a different function to SR(.) may be used to determine the modification to the position of the existing node of the virtual model. For example, during Ventricular Tachycardia (VT), the R wave is still recognizable while other waves are hard to identify; a special control method can be implemented (e.g., by replacing the function SR(.) with a different function VT(.)) so that the model can function in a way that matches this behavior without recognizing all waves. Approaches for identifying a type of cardiac functionality by processing electrical measures (e.g., sinus rhythm, VT, atrial fibrillation and so on) are well known in the art.

Thus, the value of $C_i$ may be calculated using a function that is dependent upon a cardiac functioning of the heart, e.g., as indicated in a time-series of data entries that each contain a plurality of electrical measures of the heart.

Accordingly, the position $D_I$ of a node i (as calculated in step 230, if step 260 is integrated therein) can be defined as:

$$D_i = T_i + C_i + P_i \tag{4}$$

Approaches for calculating $T_i$ have been previously described, e.g., in equations (1) and (2).

While the localized manipulation $T_i$ provides acute information, the cardiac modification is also important for providing general information on the heart's condition, it can show pathologies such as atrial fibrillation, ventricular tachycardia, ventricular fibrillation, etc.

By controlling the movement of the virtual model based on the measures of electrical activity, each part of the virtual model will move accordingly in real time. This provides additional information that was not previously available or apparent to a clinician looking at the ECG traces alone.

As indicated in equations (2) and (3), the value of $T_i$ and $C_i$ may be weighted or modified using appropriate weighting parameters $k_t$, $k_C$. In some examples, the values of $k_t$ and $k_c$ of $T_i$ are controlled or configured such that the value of $k_t$ rises as the value of $k_c$ falls, and vice versa. In particular, the value of $k_t$ may rise with increasing proximity of the part of the heart represents node i to an expected or known position of an electrode.

The proposed approach provides a mechanism for calculating the movement of positions or locations on the virtual model, particularly a movement of nodes of the virtual model. This includes information on how to compensate if there is no electrical measure associated with a particular position of the virtual model.

In step 240, providing a visual representation of the virtual model at an output user interface may comprise visually distinguishing the visual representation of different portions of the heart model, associated with different electrical measures, from one another. This can be achieved, for instance, using different colors, intensities, contrasts and/or any other visually identifiable feature.

In step 240, providing a visual representation of the virtual model at an output user interface may comprise visually distinguishing the visual representation of any part of the heart model that contains a portion associated with any electrical measures from any part of the heart model that contains a portion associated with no electrical measures. This helps to indicate that there are no acute signals in particular regions or areas of the heart, for improved understanding by the clinician or other observer of the visual representation.

The visual representation of the virtual model (provided in step 240) may be manipulable by a user or operator. In particular, a visual representation at a screen may be rotated, responsive to a user input at a user input interface, to allow a clinician to observe different elements or parts of the virtual model for improved understanding.

Thus, the method may further comprise a step 270 of (if present) receiving a user input identifying a desired view of the virtual model. Step 240 may comprise controlling the visual representation of the virtual model to provide the desired view of the virtual model.

If the method 200 is iteratively repeated, e.g., using a most recently available plurality of electrical measures, then a real-time movement of the virtual model can be displayed at the output user interface.

The proposed approach provides the representation of measures of electrical activity in a more intuitive and informative way than traditional ECG. While a conventional electrocardiogram shows the signals in multiple graphs with abstract lines, the proposed (dynamic) model shows the signals through movement, expansion, and contraction of the visual representation of the virtual model. Each electrical measure is represented through different regions of the model. It can provide all the information about the heart in a single model. The presentation of the data and the result of the diagnosis is visually easier to understand and faster to recognize.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method for providing a visual representation of a heart of a subject, the computer-implemented method comprising:

   receiving a plurality of different electrical measures responsive to electrical activity of the heart;
   receiving a virtual model of the heart, wherein each electrical measure is associated with a respective portion of the virtual model;
   for each electrical measure, modifying a position of the respective portion of the virtual model, with respect to the remainder of the virtual model, responsive to the electrical measure; and
   providing a visual representation of the virtual model at an output user interface.

2. The computer-implemented method of claim 1, wherein each electrical measure is an electrocardiography voltage of a different cardiography lead.

3. The computer-implemented method of claim 2, wherein each electrocardiography voltage is generated using one or more electrocardiography electrodes positioned on skin of the subject.

4. The computer-implemented method of any one of claims 1 to 3, wherein each electrical measure is a most recently available version of the electrical measure.

5. The computer-implemented method of any one of claims 1 to 4, wherein, for each electrical measure, modifying a position of the portion of the virtual model comprises:
   for each point of the virtual model in the portion of the virtual model, moving the point of the virtual model in a direction perpendicular to a surface of the virtual model at said point, wherein a distance of the movement of the point is responsive to the electrical measure.

6. The computer-implemented method of claim 5, wherein the distance of the movement of each point is responsive to a predetermined proportion of the electrical measure.

7. The computer-implemented method of any of claims 1 to 6, wherein the virtual model is a mesh model of the heart.

8. The computer-implemented method of claim 7, wherein:

the mesh model defines a plurality of nodes, each representing a different part of the heart; and
the computer-implemented further comprises, for each node in a subset of the plurality of nodes of the mesh model:

processing the plurality of different electrical measures to determine a movement of the part of heart represented by the node, which movement results from a movement of the heart indicated in the plurality of different electrical measures; and
modifying the position of the node responsive to the determined movement of said node.

9. The computer-implemented method of any of claims 1 to 8, wherein the step of providing a visual representation of the virtual model at an output user interface comprises visually distinguishing the visual representation of different portions of the heart model, associated with different electrical measures, from one another.

10. The computer-implemented method of any of claims 1 to 9, wherein the step of providing a visual representation of the virtual model at an output user interface comprising visually distinguishing the visual representation of any part of the heart model that contains a portion associated with any electrical measures from any part of the heart model that contains a portion associated with no electrical measures.

11. The computer-implemented method of any of claims 1 to 10, wherein each of the plurality of electrical measures is sampled at a same or similar point in time.

12. A computer-implemented method for providing a visual representation of a heart of a subject, the computer-implemented method comprising:

receiving a sequence of time-dependent data entries, each data entry comprising a plurality of different electrical measures responsive to electrical activity of the heart; and
for each time-dependent data entry in turn, performing an instance of the method of any one of claims 1 to 11, wherein the plurality of different electrical measures received in performing the instance of the method comprises the plurality of different electrical measures in the time-dependent data entry.

13. A computer program product comprising computer program code means which, when executed on a computing device having a processing system, cause the processing system to perform all of the steps of the computer-implemented method according to any of claims 1 to 12.

14. A processing system for providing a visual representation of a heart of a subject, the processing system being configured to:

receive a plurality of different electrical measures responsive to electrical activity of the heart;
receive a virtual model of the heart, wherein each electrical measure is associated with a respective portion of the virtual model;
for each electrical measure, modify a position of the respective portion of the virtual model, with respect to the remainder of the virtual model, responsive to the electrical measure; and
provide a visual representation of the virtual model at an output user interface.

15. An output user interface system comprising:

the processing system of claim 14; and
the output user interface.

FIG. 1

FIG. 2

300

FIG. 3

Europäisches
Patentamt

European
Patent Office

Office européen
des brevets

# EUROPEAN SEARCH REPORT

**Application Number**

EP 24 18 3378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/283687 A1 (KAMEN ALI [US] ET AL) 29 September 2016 (2016-09-29) * figures 1-5 * * paragraphs [0012] - [0044] * | 1-15 | INV. A61B5/00 A61B5/339 A61B5/24 G06T19/20 G16H50/50 A61B5/318 |
| X | US 2017/185740 A1 (SEEGERER PHILIPP [DE] ET AL) 29 June 2017 (2017-06-29) * figures 7-10 * * paragraph [0055] - paragraph [0077] * | 1-15 | |
| X | US 2016/338611 A1 (KALININ ALEXANDER [RU] ET AL) 24 November 2016 (2016-11-24) | 1-6,9-15 | |
| A | * figures 8, 9, 10 * * paragraphs [0093] - [0170] * | 7,8 | |
| X | EP 3 139 826 B1 (PEACS INVEST B V [NL]) 2 March 2022 (2022-03-02) | 1-6,9-15 | |
| A | * figures 1, 2 * * paragraphs [0010] - [0015], [0033] - [0044] * | 7,8 | |

**TECHNICAL FIELDS SEARCHED (IPC)**

A61B
G16H
G06T

The present search report has been drawn up for all claims

| Place of search | Date of completion of the search | Examiner |
|---|---|---|
| The Hague | 11 November 2024 | Athanasiadis, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 3378

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2024

| Patent document cited in search report | | Publication date | Patent family member(s) | | Publication date |
|---|---|---|---|---|---|
| US 2016283687 | A1 | 29-09-2016 | NONE | | |
| US 2017185740 | A1 | 29-06-2017 | CN | 106535741 A | 22-03-2017 |
| | | | CN | 111493819 A | 07-08-2020 |
| | | | US | 2017185740 A1 | 29-06-2017 |
| | | | US | 2019051419 A1 | 14-02-2019 |
| | | | WO | 2015153832 A1 | 08-10-2015 |
| US 2016338611 | A1 | 24-11-2016 | EP | 3092943 A1 | 16-11-2016 |
| | | | US | 2016338611 A1 | 24-11-2016 |
| EP 3139826 | B1 | 02-03-2022 | AU | 2015256723 A1 | 15-12-2016 |
| | | | CA | 2948132 A1 | 12-11-2015 |
| | | | EP | 3139826 A1 | 15-03-2017 |
| | | | JP | 2017518849 A | 13-07-2017 |
| | | | US | 2015320331 A1 | 12-11-2015 |
| | | | WO | 2015170978 A1 | 12-11-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017109662 A1

**Non-patent literature cited in the description**

- **RAFLA, SAMIR ; AMR KAMAL**. Localization of the occluded vessel in acute myocardial infarction. *J Cardiol Cardiovasc Med*, 2020, vol. 5, 029-33

- **A. UBACH ; C. ESTRUCH ; J. GARCIA-ESPINOSA**. On the interpolation of normal vectors for triangle meshes. *International Journal for Numerical Methods in Engineering*, September 2013, vol. 96 (4), 247-268